# EUROPEAN PATENT APPLICATION

(11) **EP 1 120 121 A2**
(43) Date of publication of application: **01.08.2001**
(21) Application number: 01300611.9
(22) Date of filing: 24.01.2001
(51) Int. Cl.: A61L 2/10

(54) **Ultra-violet container/closure sterilisation system**

(30) Priority: 26.01.2000 GB 0001673
(71) Applicant: Sheppard, Raymond William, Burnley, Lancashire BB1 28EX (GB)
(72) Inventor: Sheppard, Raymond William, Burnley, Lancashire BB1 28EX (GB)
(74) Representative: Geoffrey Owen & Company

(57) **Abstract**

A container/closure sterilisation system comprising one or more UV emitting, electrodeless plasma bulbs (10) arranged to be positionable at least partially within one or more containers/closures (24) to be sterilised, the bulbs being adapted to be energised by microwaves for sterilising inner surfaces of the one or more containers/closures (10) prior to packaging/filling.

## Description

The present invention is concerned with a sterilisation system for containers and/or closures which makes use of ultra-violet (UV).

Conventional container/closure sterilisation systems are common to the food and drink, dairy, healthcare and pharmaceutical industries for use in the packaging of perishable or contamination free products. The application of such equipment takes place prior to the product being packaged and closed.

These systems wet the inner surface of the container/closure, which is either plastic, glass or metal, with a chemical sterilising solution at around 2% strength. A typical chemical used is peroxide. The container/closure is normally passed under conventional ultra-violet (UV) light or hot air, which evaporates the sterilising solution prior to the container/closure being filled with product and sealed/closed.

These systems are normally constructed in stainless steel or mild steel; they are automatic in operation and designed to work inline or rotary.

The purpose of such systems is to produce a near to sterile container/closure inner surface to a log reduction of 4. This provides a very clean surface area in contact with the product, ensuring that the container/closure is free from harmful micro-organisms and is contamination-free for filling and packaging of product. This is desirable to maintain the shelf life of the product.

There are a number of disadvantages associated with these current types of system. They are large in size and have high operating and maintenance costs. They involve the costly storage and use of chemicals. There can be a wet chemical residue remaining in the container/closure used for packaging. It is possible for there to be
contamination to the product from wet chemical residue remaining on the inner surface of the container/closure. Furthermore, the use of such chemicals is of course not friendly to the environment.

In accordance with the present invention, there is provided a container/closure sterilisation system comprising one or more UV emitting, electrodeless plasma bulbs, and means for positioning the bulb or bulbs at least partially within one or more containers/closures to be sterilised, the bulb or bulbs being adapted to be energised by microwaves for sterilising inner surfaces of the one or more containers/closures prior to packaging/filling.

The use of such bulbs enables the achievement, without the use of chemicals, of a 6 log reduction of high, harmful product micro-organisms. This produces a near sterile dry inner container/closure surface for product filling, thereby removing the risk of contamination of product from wet chemical sterilisation. This feature is highly desirable for product packaging companies, as it offers them opportunities to extend the product shelf life and package natural taste enhanced products.

An additional benefit is the low operating cost (no requirement for chemicals) and its environmentally friendly potential.

The present invention thus enables the provision of an automatic dry sterilisation system that can produce containers/closures free from any chemicals and micro-organisms. The dry sterilisation system does not spoil the taste or affect the shelf life of the product being packaged and has the added advantage of low operating costs due to lack of chemicals. The system can be fabricated, for example, as a small modular system or as an integral part of product filling packaging systems or container capping systems.

No restrictions are placed on the bulb design by electrodes or the need for cables to supply electricity. The bulbs can operate as either low, medium or high-pressure bulbs and, because there are no electrodes, the bulb life is extended almost indefinitely.

Microwaves can be arranged to energise the bulbs by various methods, with direct contact or non-direct contact from the power packs. The bulbs can be of a wide variety of shapes to suit the particular application. This provides easy, cost effective integration into the filling systems or capping systems or as stand alone modular systems.

Preferably, the sterilisation system comprises at least one bulb holder having an inner wave guide which receives one end of a bulb and locates this end of the bulb coaxially within an outer waveguide.

Preferably, the bulb holder formed by the inner and outer waveguides is embraced by a screen providing microwave protection for the surrounding environment.

Preferably, the sterilisation system includes one or more cooling ports for introducing air or other gas around the bulb holder or holders for maintaining a cool operating temperature for the bulb or bulbs.

The air or other gas introduced by the ports can be of a nature that it enhances the sterilisation process and thereby enhances the shelf life of the product being packaged.

Advantageously, the sterilisation system further comprises a mechanism which enables the bulb or bulbs to be displaced from a raised standby position to a lowered position where, in use, at least part of the bulb or bulbs enters a container to be sterilised such that the whole of the interior surface or surfaces of the container are exposed to the UV light of the bulb or bulbs.

In some embodiments, there can be multiple bulbs disposed in a parallel array which can be displaced simultaneously by said mechanism to enter a plurality of containers.

In some embodiments the inner and outer waveguides are coupled by a cable to a microwave generator.

Other embodiments can comprise one or more microwave power packs for energising the bulb or bulbs, said one or more microwave power packs surrounding a tunnel or oven in which the bulb or bulbs are disposed.

The invention is described further hereinafter, by way of example only, with reference to the accompanying drawings, in which:-
Fig. 1 is a sectional side view of one embodiment of a sterilisation apparatus in accordance with the present invention, with the UV bulb positioned above a container to be sterilised; and
Fig 2 is a sectional side view of the apparatus of Fig. 1 with the UV bulb positioned so as to extend into the container to be sterilised.

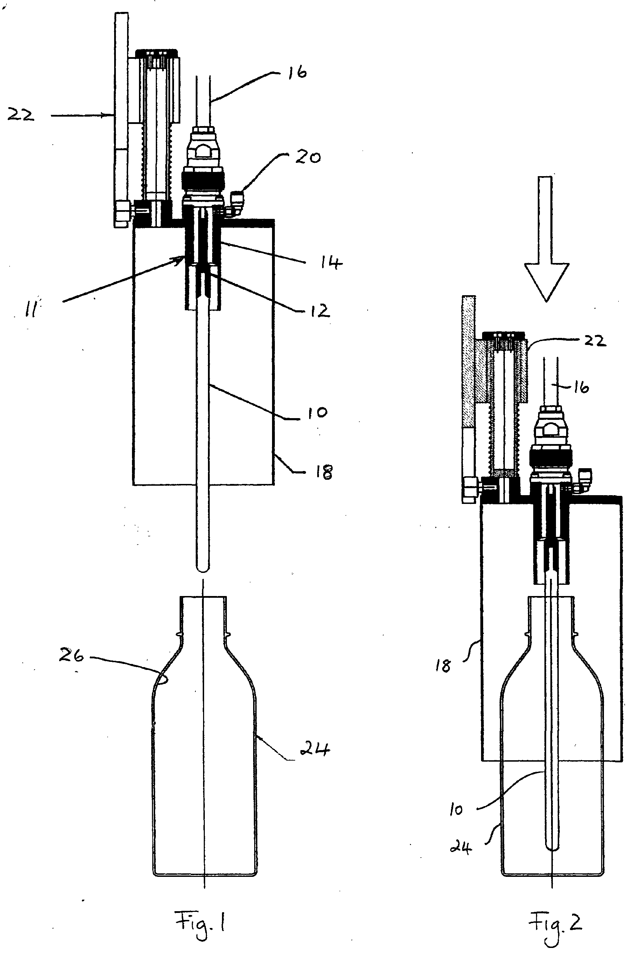

The apparatus of Figs. 1 and 2 makes use of one or more plasma bulbs 10 (only one being illustrated) of the type which are energised by microwaves without the need for electrodes. The bulb 10 in figures 1 and 2 is located by a bulb holder 11 comprising an inner wave guide 12 which receives one end of the bulb and locates this end of the bulb coaxially within an outer wave guide 14. The inner and outer wave guides 12, 14 are coupled by a cable 16 to a microwave generator (not shown). The bulb holder formed by the inner and outer wave guides 12, 14 is embraced by a cylindrical screen 18 providing microwave protection for the surrounding environment. Air or other gas can be introduced via cooling ports 20 for maintaining a cool operating temperature for the bulb 10. The air or other gas introduced via the ports 20 can also be chosen such as to enhance the sterilisation process and thereby enhance the shelf life of the product being packaged.

The bulb holder and bulb held thereby are attached to a mechanism 22 that enables the bulb to be displaced (vertically in this instance) from a raised, standby position shown in figure 1 to a lowered position shown in figure 2 where at least part of the bulb 10 enters a container 24 to be sterilised such that the whole of the interior surface(s) 26 of the container 24 are exposed to the UV light of the bulb 10. It is of course possible in an alternative arrangement for the bulb 10 to remain fixed and for the container(s) 24 to be moved upwardly to achieve the figure 2 condition or indeed for both the bulb and container to be moved. The introduction of the bulb into the container provides the capability for sterilisation of the inner surface of the container at high volume. For example, there can be multiple bulbs 10 disposed in a parallel array which can be caused simultaneously to enter a plurality of containers 24.

A number of different bulbs 10 are available for use in this system to provide the desired output rate during operations. The design and operation can be inline, rotary or parallel and is preferably, fabricated either in stainless steel or mild steel as modular units or as an integral part of a product filling and packaging machine.

Whereas we have referred above to the apparatus being used for sterilising "containers", it may also be used to sterilise other product receiving structures, such as closure structures for containers.

The design of the plasma bulbs 10 can take many shapes and sizes to suite the shape of the container/closure. This ensures that the UV light irradiates all inner surfaces of the container/closure.

One or more microwave power packs are needed to energise the bulb(s), and can be located close by by way of power cables 16, linking the power pack to the bulbs. Alternatively, the power packs can surround the bulbs in a tunnel or oven arrangement. This latter method of operation would not require the use of cables to energise the bulbs, e.g. the bulbs only need to be sited in the tunnel or oven for exposure to microwaves to energise the plasma bulbs.

In the latter case, the outsides of the containers/closures are also sterilised by passing them through the tunnel or placing them in an oven.

## Claims

1. A container/closure sterilisation system characterised by one or more UV emitting, electrodeless plasma bulbs (10), and means for positioning said one or more UV emitting, electrodeless plasma bulbs (10) at least partially within one or more containers/closures (24) to be sterilised, the bulb or bulbs (10) being adapted to be energised by microwaves for sterilising inner surfaces of the one or more containers/closures (24) prior to packaging/filling.

2. A sterilisation system as claimed in claim 1, comprising at least one bulb holder (11) having an inner wave guide (12) which receives one end of a bulb (10) and locates this end of the bulb coaxially within an outer waveguide (14).

3. A sterilisation system as claimed in claim 2 wherein the bulb holder (11) formed by the inner and outer waveguides (12, 14) is embraced by a screen (18) providing microwave protection for the surrounding environment.

4. A sterilisation system as claimed in claim 2 or 3 comprising one or more cooling ports (20) for introducing air or other gas around the bulb holder or holders (11) for maintaining a cool operating temperature for the bulb or bulbs (10).

5. A sterilisation system as claimed in claim 4, wherein the air or other gas introduced by the ports (20) is of a nature that it enhances the sterilisation process and thereby enhances the shelf life of the product being packaged.

6. A sterilisation system as claimed in any of claims 2 to 5, further comprising a mechanism (22) which enables the bulb or bulbs (10) to be displaced from a raised standby position to a lowered position where, in use, at least part of the bulb or bulbs (10) enters a container (24) to be sterilised such that the whole of the interior surface or surfaces (26) of the container (24) are exposed to the UV light of the bulb or bulbs (10).

7. A sterilisation system as claimed in claim 6, in which there are multiple bulbs (10) disposed in a parallel array which can be displaced simultaneously by said mechanism (22) to enter a plurality of containers (24).

8. A sterilisation system as claimed in any of claims 2 to 7, wherein said inner and outer waveguides (12, 14) are coupled by a cable (16) to a microwave generator.

9. A sterilisation system as claimed in any of claims 2 to 7, further comprising one or more microwave power packs for energising the bulb or bulbs (10), said one or more microwave power packs surrounding a tunnel or oven in which the bulb or bulbs are disposed.
